# EUROPÄISCHE PATENTSCHRIFT

(11) **EP 2 347 707 B1**
(45) Veröffentlichungstag und Bekanntmachung des Hinweises auf die Patenterteilung: **15.07.2015**
(21) Anmeldenummer: 10151429.7
(22) Anmeldetag: 22.01.2010
(51) Int. Cl.: A61B 5/022

(54) **Schlauch für eine Blutdruckmanschette, Blutdruckmanschette und Verfahren**
Tube for a blood pressure cuff, blood pressure cuff and method
Tuyau pour un tensiomètre, tensiomètre et procédé

(43) Veröffentlichungstag der Anmeldung: 27.07.2011
(73) Patentinhaber: Dräger Medical GmbH, 23558 Lübeck (DE)
(72) Erfinder: Dr. Grassl, Thomas, 23560, Lübeck (DE)

(56) Entgegenhaltungen:
- EP-A1- 0 337 162
- EP-A1- 1 614 451
- JP-A- 2004 223 102
- JP-A- 2009 072 548
- US-A- 6 095 983

## Beschreibung

Aus der Praxis sind Blutdruckmanschetten bekannt, die zu ihrem Aufblasen einen Schlauch aufweisen.

Die JP 2004-223102 A, von der die vorliegende Erfindung ausgeht, offenbart einen Schlauch für eine Blutdruckmanschette zum Messen des Blutdrucks eines Patienten an einer seiner Extremitäten. Der Schlauch weist einen Querschnitt auf, wobei die Innenfläche und die Außenfläche des Schlauchs unterschiedliche Formen aufweisen. Die Außenfläche des Schlauchs ist im Querschnitt kreisförmig. Die Innenfläche des Schlauchs hingegen weist im Querschnitt eine ungerade Anzahl von geraden Abschnitten sowie von abgewinkelten beziehungsweise abgerundeten Abschnitten auf, welche die geraden Abschnitte verbinden, um ein zu starkes Biegen oder Abknicken des Schlauchs und in der Folge ein Blockieren des Schlauchs zu verhindern. Somit weist die Innenfläche des Schlauchs im Querschnitt mehrere Abschnitte mit unterschiedlicher Krümmung auf. Die Außenfläche des Schlauchs weist im Querschnitt jedoch eine gleichmäßige Krümmung auf, die nicht mit der Krümmung der Innenfläche übereinstimmt.

Aus der U.S. 6,095,983 ist ein Schlauch für eine Blutdruckmanschette zum Messen des Blutdrucks eines Patienten bekannt, deren einer, von der Blutdruckmanschette entfernter Teil zwei im Querschnitt symmetrisch zueinander gebildete Röhren - eine zur Aufnahme von elektrischen Leitungen und die andere zur Aufnahme und zum Leiten eines Fluids - aufweist. Beide Röhren sind integral gebildet, wobei die gemeinsame Außenfläche beider Röhren unterschiedliche Krümmungen, ähnlich einer Acht, aufweist. Der Endabschnitt eines Schlauchs für das Fluid, der an die Blutdruckmanschette angebracht ist, ist sowohl in Bezug auf die Außenfläche als auch in Bezug auf die Innenfläche kreisförmig.

Die JP 2009-72548 A lehrt einen Schlauch für eine Blutdruckmanschette zum Messen des Blutdrucks eines Patienten an einer Extremität. Der Schlauch weist im Querschnitt entlang seiner Außenfläche auf gegenüberliegenden Seiten Verdickungen zur Verstärkung des Schlauches gegen ein Abknicken auf, so dass die Außenfläche des Schlauches im Querschnitt verschiedene Abschnitte mit unterschiedlicher Krümmung aufweist. Die Innenfläche des Schlauches weist jedoch einen kreisförmigen Querschnitt, das heißt eine gleichmäßige Krümmung, auf.

Eine Aufgabe der vorliegenden Erfindung ist es, eine Kombination aus einer Blutdruckmanschette und einem Schlauch bereitzustellen, die bei einer Blutdruckmessung an der Extremität eines Patienten, insbesondere eines Neonaten, keine unerwünschten Druckstellen oder Einschnürungen auf der Haut des Patienten hervorruft und die eine möglichst fehlerfreie Messung des Blutdrucks zulässt..

Diese Aufgabe der vorliegenden Erfindung wird durch eine Kombination aus einer Blutdruckmanschette und einem Schlauch mit den Merkmalen des Anspruchs 1 gelöst.

Der erfindungsgemäße Schlauch ist vorzugsweise luft- oder gasdicht und elastisch.

Der erfindungsgemäße Schlauch weist in wenigstens einem Abschnitt hiervon einen Querschnitt auf, welcher wenigstens zwei, voneinander verschieden stark oder unterschiedlich gekrümmte Querschnittsabschnitte aufweist.

Vorteilhafte Weiterbildungen sind jeweils Gegenstand von Unteransprüchen und Ausführungsformen. Dabei können erfindungsgemäße Ausführungsformen (in beliebigen Kombinationen) eines oder mehrere Merkmale aufweisen oder hieraus bestehen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen.

Eine "Extremität eines Patienten" bezeichnet dessen Arme und Beine.

Eine "Blutdruckmanschette" bezeichnet vorliegend eine vom Arzt mitführbare aufblasbare Einrichtung, welche vorgesehen und ausgestaltet ist, um einen Blutdruck nicht-invasiv zu messen.

In einer erfindungsgemäßen Ausführungsform liegen die wenigstens zwei, voneinander verschieden stark oder unterschiedlich gekrümmten Querschnittsabschnitte sowohl in einem ersten Zustand des Nicht-Gebrauchens als auch in einem zweiten Zustand des Messens jeweils verschieden stark oder unterschiedlich gekrümmt vor.

Der Begriff "erster Zustand des Nicht-Gebrauchens", wie er hierin verwendet wird, bezeichnet einen ersten Zustand des Schlauchs, in welchem der Schlauch nicht zum Messen benutzt wird und auch nicht an der Extremität angelegt ist. Der Zustand des Nicht-Gebrauchens kann ein Zustand des Schlauchs sein, in welchem der Schlauch keinerlei äußeren Krafteinwirkungen und/oder Formänderungen unterliegt.

Der Begriff "zweiter Zustand des Messens", wie er hierin verwendet wird, bezeichnet einen zweiten Zustand des Schlauchs, in welchem der Schlauch zum Messen eines Blutdrucks zum Einsatz kommt, insbesondere durch Luft, oder Gas allgemein, in seinem Inneren mit Druck beaufschlagt ist. Der zweite Zustand kann einen Zeitpunkt oder Moment beschreiben, in welchem mittels der Blutdruckmanschette der systolische oder der diastolische Druck gemessen wird.

In bestimmten Ausführungsformen der vorliegenden Erfindung kann der Schlauch durchgehend bzw. über seine gesamte Länge den gleichen Querschnitt mit den vorstehend angegebenen erfindungsgemäßen Merkmalen aufweisen. Dies kann beispielsweise vorteilhaft ermöglichen, den Schlauch fertigungs- und kostengünstig als Meterware herzustellen.

In anderen Ausführungsformen der vorliegenden Erfindung weist der Schlauch nur in einem Abschnitt einen Querschnitt mit den vorstehend angegebenen erfindungsgemäßen Merkmalen auf.

Ein solcher Abschnitt ist ein Endabschnitt des Schlauchs. Der Abschnitt des Schlauchs ist dazu vorgesehen, mit der Blutdruckmanschette verbunden zu werden. Der Abschnitt des Schlauchs ist mit der Blutdruckmanschette verbunden.

Der Abschnitt ist in einer erfindungsgemäßen Ausführungsform ein Manschettenanschluss.

Der gesamte Schlauch ist in einer erfindungsgemäßen Ausführungsform ein Manschettenanschluss.

Der Manschettenanschluss ist in einer erfindungsgemäßen Ausführungsform ein separates Anschlussstück. Der als Manschettenanschluss ausgestaltete erfindungsgemäße Schlauch kann mit einem weiteren Schlauch zur Herstellung einer Blutdruckmanschette verbindbar oder verbunden sein. Der Manschettenanschluss kann kraft- und/oder form- und/oder stoffschlüssig mit dem erfindungsgemäßen Schlauch oder dem anderen Schlauch verbunden sein.

Der Begriff "Querschnitt", wie er hierin verwendet wird, bezeichnet in einer erfindungsgemäßen Ausführungsform eine räumliche Ausdehnung des Schlauchs bzw. des Schlauchmaterials in einer Richtung quer, d.h. in einem Winkel von im Wesentlichen oder exakt 90°, zu einer Längsrichtung des gestreckten Schlauchs.

Der erfindungsgemäße Schlauch weist in einer erfindungsgemäßen Ausführungsform neben dem oben beschriebenen Querschnitt einen weiteren, sich hiervon unterscheidenden Querschnitt auf. In dem weiteren Querschnitt können andere Krümmungsverhältnisse als im oben beschriebenen Querschnitt vorliegen. Der weitere Querschnitt kann insbesondere rund ausgestaltet sein. Der Begriff "Querschnittsabschnitt", wie er hierin verwendet wird, bezeichnet einen Abschnitt des erfindungsgemäßen Schlauchs mit einem wie oben beschriebenen Querschnitt.

In einer weiteren Ausführungsform weist der erfindungsgemäße Schlauch neben den zwei unterschiedlich gekrümmten Querschnittsabschnitten weitere Querschnittsabschnitte auf, weiche wiederum eigene, beliebige Krümmungen aufweisen können.

Der Begriff "Krümmung", wie er hierin verwendet wird, bezeichnet eine Abweichung der Erstreckung einer Linie oder einer (gekrümmten) Fläche von einer Geraden oder einer Ebene. Dabei ist zu beachten, dass auch eine Gerade eine Krümmung (mit Radius 0) im Sinne der Erfindung aufweist.

Unter einer Krümmung wird erfindungsgemäß auch eine Abwinkelung verstanden. So weist beispielsweise ein Querschnitt in Gestalt eines gleichwinkeligen Dreiecks im Sinne der Erfindung zwei Querschnittsabschnitte mit unterschiedlicher Krümmung auf: die Winkel zwischen den Schenkeln (wobei sich die Winkel aufgrund der Gleichwinkeligkeit nicht voneinander unterscheiden) und die Krümmung der Schenkel selber, wobei deren Krümmung wie oben ausgeführt aufgrund ihrer geraden Erstreckung im Sinne der Entscheidung als eine Krümmung mit Radius 0 verstanden wird. Ein Querschnitt in Form eines gleichschenkeligen Dreiecks könnte im Sinne der Erfindung folglich zwei oder entsprechend mehr als zwei unterschiedlich gekrümmte Querschnittsabschnitte aufweisen.

Die unterschiedliche Krümmung der einzelnen oder der betrachteten Querschnittabschnitte kann sich auf die Krümmungen der Außenfläche und/oder der Innenflächen des Schlauchs in Höhe des betrachteten Querschnitts beziehen.

Der Begriff "unterschiedlich gekrümmt", wie er hierin verwendet wird, meint, dass wenigstens zwei Querschnittsabschnitte des Querschnitts des Schlauchs eine unterschiedliche Krümmung aufweisen, insbesondere in unterschiedlichem Ausmaß gekrümmt sind.

In einer Ausführungsform der vorliegenden Erfindung weist ein beim Messen der Extremität zugewandter Querschnittsabschnitt des Schlauchs im ersten Zustand des Nicht-Gebrauchens eine Krümmung von im Wesentlichen oder gleich 0 Grad oder eine, konkave Krümmung auf - letzteres betrachtet basierend auf seinem späteren Anliegen an einer Extremität - von einem Mittelpunkt der Extremität.

Ein Querschnittsabschnitt mit einer Krümmung von 0 Grad ist ein geradliniger Querschnittsabschnitt des Schlauchs.

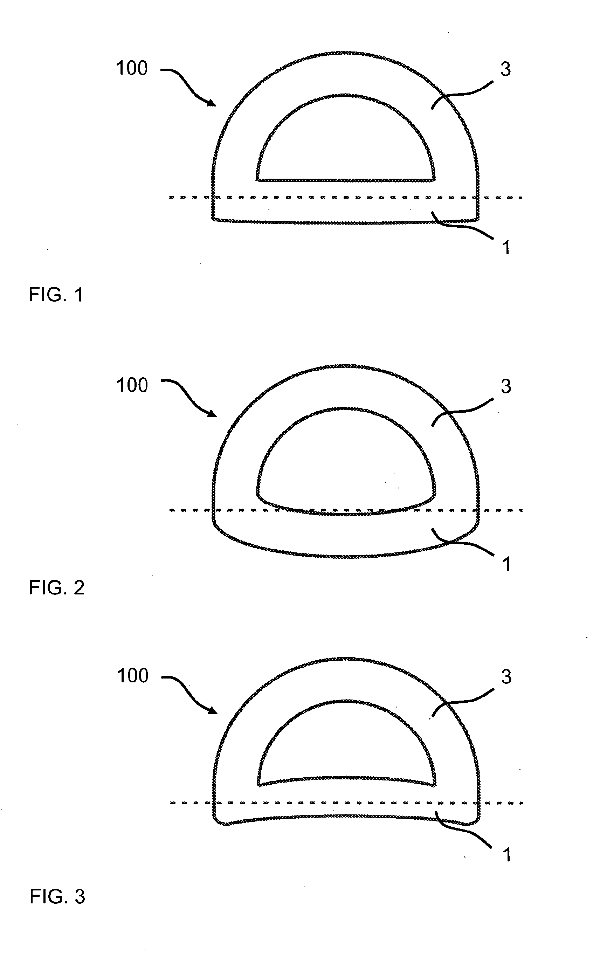

Der Schlauch weist in einer erfindungsgemäßen Ausführungsform im ersten Zustand des Nicht-Messens einen geschlossenen, im Wesentlichen oder annähernd halbkreisförmigen Querschnitt auf. Ein solcher Querschnitt ist beispielhaft in der beigefügten Fig. 1 gezeigt.

In einer solchen Ausgestaltung weist ein Querschnittsabschnitt des Schlauchs eine geradlinige Form, d.h. eine Krümmung von im Wesentlichen oder vollständig 0 Grad, ein anderer z.B. eine bogen- oder sichelartige Form mit einer von 0 Grad verschiedenen Krümmung auf.

In einer weiteren Ausführungsform weist ein der Extremität zugewandter Querschnittsabschnitt des Schlauchs im ersten Zustand des Nicht-Gebrauchens eine - bezogen auf die Extremität des Patienten, an welcher dieser Querschnittsabschnitt zu einem späteren Zeitpunkt zum Zwecke des Messen zum Liegen kommen soll - konvexe Krümmung auf, wie dies beispielsweise in Fig. 2 dargestellt ist.

Erfindungsgemäßweist der der Extremität zugewandte Querschnittsabschnitt des Schlauchs im zweiten Zustand des Messens eine Krümmung von im Wesentlichen oder gleich 0 Grad oder eine, bezogen auf die Extremität, konkave Krümmung auf.

Der Begriff "bezogen auf die Extremität konkave Krümmung", wie er hierin verwendet wird, bezeichnet eine der Extremität zugewandte Krümmung des einen Querschnittsabschnitts des Schlauchs derart, dass sich der konkav gekrümmte Abschnitt der Extremität mehr oder weniger anschmiegt - im Gegensatz zu einer bezogen auf die Extremität konvexen Krümmung.

In einer Ausführungsform der vorliegenden Erfindung weist die konkave Krümmung im zweiten Zustand des Messens einen Radius r in einem Bereich von r zwischen einschließlich 0,5 cm bis einschließlich 2 cm auf. Der Mittelpunkt und damit der Ausgangspunkt für die Bestimmung des Radius liegt dabei in der Extremität, nicht außerhalb dieser.

Beispielsweise kann eine Krümmung, berechnet nach der Formel 1/r, Werte von zwischen einschließlich 0,5 cm⁻¹ bis einschließlich 2 cm⁻¹ aufweisen.

In einer weiteren Ausführungsform der vorliegenden Erfindung weist der Schlauch im zweiten Zustand des Messens einen, bezogen auf die Extremität, sichelförmigen, geschlossenen Querschnitt auf.

Der Begriff "sichelförmiger Querschnitt", wie er hierin verwendet wird, meint, dass beide Querschnittsabschnitte des Querschnitts, bezogen auf die Extremität, konkav ausgestaltet sein. Ein derart sichelförmiges Aussehen des Querschnitts des Schlauchs kann beispielsweise der beigefügten Fig. 2 der Zeichnung entnommen werden.

In einer weiteren Ausführungsform des erfindungsgemäßen Schlauchs weist ein einem äußeren Umfang der Extremität des Patienten im zweiten Zustand des Messens des Blutdrucks zugewandter Querschnittsabschnitt eine der Extremität zugewandte konkave Krümmung in einem Bereich von 0 bis 1/r auf, wobei r jeweils der Radius eines im Bereich eines Abschnitts des äußeren Umfangs der Extremität angelegten Schmiegekreises ist.

Der Begriff "Schmiegekreis", wie er hierin verwendet wird, bezeichnet einen Kreis oder Krümmungskreis, welcher im Bereich eines Abschnitts des äußeren Umfangs der Extremität gelegt wurde und sich am besten an einen Punkt auf dem der Extremität zugewandten Querschnittsabschnitt des Schlauchs annähert.

Je nach Beschaffenheit, wie zum Beispiel Lage oder Krümmung einer unmittelbaren oder näheren Umgebung des Punktes auf dem der Extremität zugewandten Querschnittsabschnitt des Schlauchs, kann der im Bereich eines Abschnitts des äußeren Umfangs der Extremität angelegte Schmiegekreis variieren. So kann er insbesondere entsprechend verschiedene Radien und/oder Kreismittelpunkte, d.h. Krümmungsmittelpunkte, aufweisen.

Da die Krümmung in der vorliegenden Ausführungsform an diskreten Punkten auf dem der Extremität zugewandten Querschnittsabschnitt des Schlauchs betrachtet wird, können diskrete Werte für die Krümmung stark variieren. Zum Beispiel kann der der Extremität zugewandte Querschnittsabschnitt des Schlauchs lokal möglicherweise konvex gekrümmt sein.

Die gesamte Krümmung des der Extremität zugewandten Querschnittsabschnitts des Schlauchs kann sich aus der Integralbildung aller im Bereich des der Extremität zugewandten Querschnittsabschnitts des Schlauchs diskreten Krümmungen ergeben.

Der erfindungsgemäße Schlauch ist in einer erfindungsgemäßen Ausführungsform aus einem Thermoplast gefertigt oder weist ein solches Material auf. Der erfindungsgemäße Schlauch weist in einer erfindungsgemäßen Ausführungsform ein Polyurethan auf oder besteht hieraus. In einer erfindungsgemäßen Ausführungsform ist der erfindungsgemäße Schlauch aus einem thermoplastischen Polyurethan, insbesondere aus TPR 60 ShA (Prüfverfahren shore A) gefertigt. Es sind aber auch andere thermoplastische Elastomere, PVC oder Silikon vorstellbar und von der Erfindung umfasst.

In bestimmten Ausführungsformen der vorliegenden Erfindung weist der erfindungsgemäße Schlauch in wenigstens einem Abschnitt hiervon oder über seine gesamte Länge einen Außendurchmesser von kleiner als 8 mm, vorzugsweise 5 mm auf. Der Begriff "Außendurchmesser", wie er hierin verwendet wird, kann sich in manchen erfindungsgemäßen Ausführungsformen auf einen Gesamtdurchmesser eines im Wesentlichen symmetrisch ausgestalteten Schlauchs, wie einer runden Form oder Kontur des Schlauchs, beziehen. Der Begriff kann sich auf die maximale oder durchschnittliche Erstreckung in einer Querschnittsrichtung beziehen.

In einer weiteren Ausführungsform der vorliegenden Erfindung weist der Schlauch wenigstens einen Manschettenanschluss auf oder ist als ein solcher ausgestaltet. Der Manschettenanschluss liegt beim Messen des Blutdrucks wenigstens abschnittsweise oder vollständig in einem Inneren der Blutdruckmanschette und unterliegt der Druckerhöhung durch die Blutdruckmanschette.

Der Schlauch kann im Bereich eines Manschettenanschlusses wenigstens abschnittsweise kraft- und/oder form- und/oder stoffschlüssig mit der Blutdruckmanschette verbunden sein.

Eine erfindungsgemäße Blutdruckmanschette kann wenigstens eine erste Folie und eine zweite Folie sowie wenigstens einen erfindungsgemäßen Schlauch aufweisen, welcher wenigstens abschnittsweise - im Bereich des Manschettenanschlusses - zwischen die erste Folie und die zweite Folie der Blutdruckmanschette eingeschweißt ist.

Die an der Innenseite und/oder der Außenseite der Blutdruckmanschette angeordneten Folien können aus PU (Polyurethan) oder PVC gebildet sein oder wenigstens abschnittsweise Polyurethane aufweisen. An der Innenseite kann die Blutdruckmanschette ferner wenigstens abschnittsweise beschichtetes Velours aufweisen. Während des zweiten Zustands des Messens kann die Innenseite an der Extremität des Patienten anliegen.

Die Blutdruckmanschette kann abgesehen vom verwendeten Schlauch eine herkömmliche Blutdruckmanschette sein, wie sie einem Fachmann zum Messen des Blutdrucks eines Patienten bekannt ist.

Die erfindungsgemäße Blutdruckmanschette kann hergestellt werden, indem der erfindungsgemäße Schlauch, insbesondere in einem Manschettenanschluss desselben, zwischen der Folie an der Innenseite und der Folien an der Außenseite der Blutdruckmanschette angeordnet und mit den Folien kraft- und/oder form- und/oder stoffschlüssig verbunden wird.

Zum Beispiel kann der Schlauch im Bereich des Manschettenanschlusses mittels eines Hochfrequenz - Schweißverfahrens (HF - Schweißverfahrens) mit und/oder zwischen den beiden Folien verbunden werden.

Im Bereich der Innenseite der Blutdruckmanschette kann der Manschettenanschluss des Schlauchs im ersten Zustand des Nicht-Gebrauchens eine gerade Fläche, d.h. eine Krümmung von im Wesentlichen oder exakt 0 Grad, aufweisen.

Dabei wird eine Blutdruckmanschette gemäß der vorliegenden Erfindung hergestellt, wobei wenigstens ein Schlauch gemäß der vorliegenden Erfindung verwendet wird.

Die vorliegende Erfindung stellt eine Kombination aus einem Schlauch und einer Blutdruckmanschette bereit, welche vorteilhaft eine gute Übertragung des Messsignals' sicherstellen kann.

Beim Messen ist der der Extremität zugewandte Querschnittsabschnitt gerade oder hat eine konkav gewölbte Fläche. Damit kann vorteilhaft sichergestellt werden, dass der Schlauch oder sein Manschettenanschluss vor allem bei Messungen am Arm eines Neonaten mit naturgemäß sehr kleinem Durchmesser zu keinen Messartefakten führt oder zum Zwecke einer korrekten Messung nach Erkennen der Messartefakte abgenommen und erneut angelegt werden muss. Daneben kann vorteilhaft ein punktuelles Einwirken der Blutdruckmanschette während des Messens vermieden werden.

Mit dem erfindungsgemäßen Schlauch kann es vorteilhaft möglich sein, ein ungünstiges Verhältnis zwischen dem Schlauchdurchmesser und den Durchmessern von Arterie und Extremität beim Messen des Blutdrucks zu vermeiden, wie es vor allem beim Messen an Früh- oder Neugeborenen zu beobachten ist. Dieses ungünstige Verhältnis kann, wie der Erfinder feststellen konnte, bei relativ kleiner Aufdruckfläche des Schlauchs beim Messen auf z.B. den Oberarm eines Neonaten dazu führen, dass eine Kompression der Oberarmarterie bereits durch das Drücken des Schlauchs auf die Arterie erfolgt, und dass die Arterie nicht - wie beim Blutdruckmessen mittels Blutdruckmanschette gefordert - durch den Druck der Blutdruckmanschette selber. Da die Aufdruckfläche des Schlauchs auf den Oberarm und damit auch auf dessen Hauptarterie bei Einsatz des erfindungsgemäßen Schlauchs aufgrund dessen geometrischer Ausgestaltung deutlich größer ist, besteht das oben beschriebene ungünstige Flächenverhältnis nicht. Die erzielten Messergebnisse sind damit zuverlässiger als jene, die mit Blutdruckmanschetten erzielt werden, deren Schläuche ein anderes Querschnittprofil als der erfindungsgemäße Schlauch aufweisen.

Der erfindungsgemäße Schlauch kann aufgrund seines wie oben beschriebenen besonderen Querschnittprofils derart an der Blutdruckmanschette befestigt sein, dass zumindest Abschnitte hiervon auch in einem Inneren der Blutdruckmanschette vorliegen können. Hierdurch kann vorteilhaft ein Abknicken des Schlauchs wie es bei Schläuchen des Standes der Technik, welche nicht bis in das Innere der Blutdruckmanschette reichen, auftreten kann, vorteilhaft vermieden werden.

Daneben kann es vorteilhaft möglich sein, eine Dämpfung des Messsignals zu verhindern.

In manchen erfindungsgemäßen Ausführungsformen ist es ferner vorteilhaftmöglich, den lokal, vor allem durch den Schlauch, aufgebrachten Druck auf den Arm derart zu verringern, dass es nicht zu Druckstellen oder anderen Beeinträchtigungen von Gewebe kommt. Ein abschnittsweises Einschnüren der Extremität kann vorteilhaft vermieden werden.

Ferner kann es bei Verwenden des erfindungsgemäßen Schlauchs möglich sein, den Manschettenanschluss auch direkt am Anfang der Blutdruckmanschette zu positionieren, ohne dass dabei eine Versteifung der Manschette auf einer relativ großen Fläche erfolgt wie bei Lösungen des Standes der Technik, bei welchen Schlauchnippel als 90°-Winkel auf der Außenseite der Blutdruckmanschette angeschweißt sind. Auf diese Weise kann es wiederum vorteilhaft möglich sein, eine Verringerung des zur Messung erforderlichen Querschnitts des Armes vor allem des Neonaten und/oder dessen Deformation aufgrund der Versteifung zu verhindern.

Im Folgenden wird die vorliegende Erfindung beispielhaft unter Bezugnahme auf die beigefügte Zeichnung beschrieben. Es zeigt:
- Fig. 1: einen Querschnitt eines erfindungsgemäßen Schlauchs in einem ersten Zustand des Nicht-Gebrauchens gemäß einer ersten Ausführungsform;
- Fig. 2: einen Querschnitt eines erfindungsgemäßen Schlauchs in einem ersten Zustand des Nicht-Gebrauchens gemäß einer zweiten Ausführungsform;
- Fig. 3: einen Querschnitt eines erfindungsgemäßen Schlauchs im zweiten Zustand des Messens;
- Fig. 4: einen Querschnitt durch eine erfindungsgemäße Blutdruckmanschette während eines zweiten Zustand des Messens an einer Extremität;
- Fig. 5: eine Draufsicht auf eine erfindungsgemäße Blutdruckmanschette in einem ersten Zustand des Nicht-Gebrauchens;
- Fig. 6: einen Querschnitt eines erfindungsgemäßen Schlauchs in einem ersten Zustand des Nicht-Gebrauchens gemäß einer dritten Ausführungsform; und
- Fig. 7: einen Querschnitt eines erfindungsgemäßen Schlauchs in einem ersten Zustand des Nicht-Gebrauchens gemäß einer vierten Ausführungsform.

Fig. 1 zeigt einen Querschnitt eines erfindungsgemäßen Schlauchs 100 in einem ersten Zustand des Nicht-Gebrauchens gemäß einer ersten Ausführungsform.

Der Schlauch 100 weist einen (in seinem späteren Gebrauch) der Extremität (in Fig. 1 nicht gezeigt) zugewandten Querschnittsabschnitt 1 und einen der Extremität abgewandten Querschnittsabschnitt 3 auf, die zusammen einen geschlossenen Halbkreis darstellen.

Der der Extremität zugewandte Querschnittsabschnitt 1 weist eine Krümmung von im Wesentlichen 0 Grad auf. Der der Extremität abgewandte Querschnittsabschnitt 3 weist eine in Bezug auf die Extremität, an welcher der Schlauch beim Vorgang des Messens zu liegen kommen soll, konkave Form auf.

Damit sind der abgewandte und der zugewandte Querschnittsabschnitt 1, 3 - im Vergleich zueinander - jeweils unterschiedlich stark gekrümmt.

Fig. 2 zeigt einen Querschnitt eines erfindungsgemäßen Schlauchs in einem ersten Zustand des Nicht-Gebrauchens gemäß einer zweiten Ausführungsform.

Der der Extremität zugewandte Querschnittsabschnitt 1 weist hierbei eine bezogen auf die Extremität (leicht) konvexe Krümmung auf.

Damit sind der abgewandte und der zugewandte Querschnittsabschnitt 1, 3 - im Vergleich zueinander - wiederum jeweils unterschiedlich gekrümmt.

Fig. 3 zeigt einen Querschnitt eines erfindungsgemäßen Schlauchs in einem zweiten Zustand des Messens.

Der der Extremität zugewandte Querschnittsabschnitt 1 weist eine bezogen auf die Extremität konkave Krümmung auf. Der der Extremität abgewandte Querschnittsabschnitt 3 weist in Bezug auf die Extremität ebenfalls eine konkave Form auf.

Der geschlossene Querschnitt des Schlauchs 100 weist somit eine in Bezug auf die Extremität konkave, insgesamt sichelförmige Form oder Kontur auf.

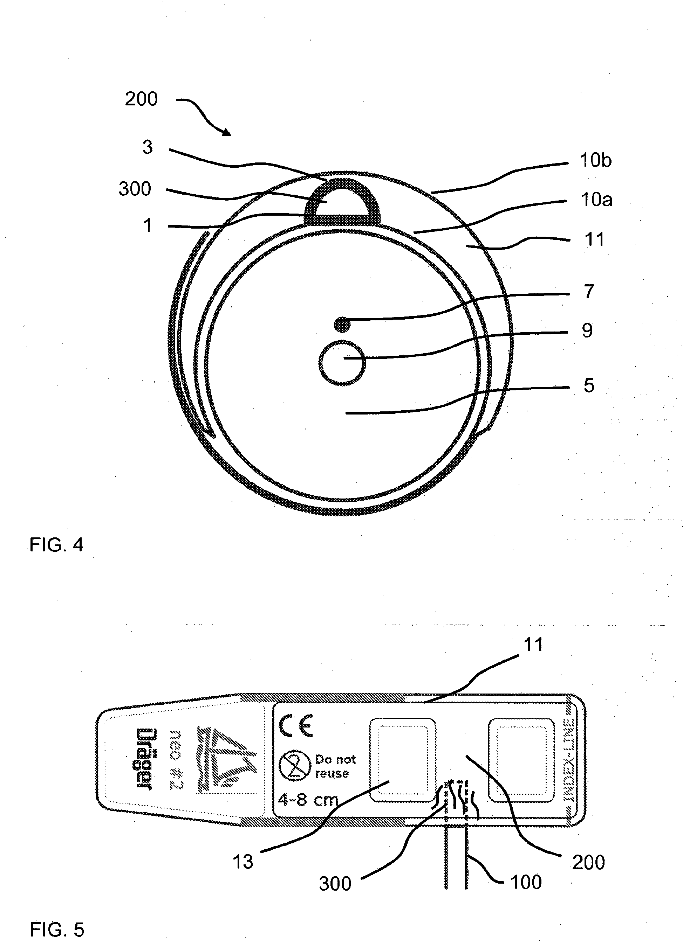

Fig. 4 zeigt einen Querschnitt durch eine erfindungsgemäße Blutdruckmanschette 200 während eines zweiten Zustands des Messens an einer Extremität.

Die Blutdruckmanschette 200 ist geschlossen um die Extremität, z.B. einen Arm 5 mit einer Arterie 7 und einem Oberarmknochen 9 gelegt.

Die Blutdruckmanschette 200 weist den als einen Manschettenanschluss 300 ausgestalteten erfindungsgemäßen Schlauch 100 auf. Wie oben diskutiert, kann der erfindungsgemäße Schlauch 100 wahlweise einen Manschettenabschluss 300 mit den erfindungsgemäßen Merkmalen der unterschiedlich gekrümmten Querschnittsabschnitte 1, 3 aufweisen, oder aber durchgehend, also nicht nur im Bereich seines Manschettenanschlusses 300, mit unterschiedlich gekrümmten Querschnittsabschnitten 1, 3 ausgestaltet sein.

Wie in Fig. 4 (und auch in Fig. 5) gezeigt ist, ist der Manschettenanschluss 300 in einem Endbereich der Blutdruckmanschette 200 angeordnet.

Wie in Fig. 4 gut zu erkennen ist, weist der Querschnitt des Manschettenanschlusses 300 während des zweiten Zustands des Messens eine sichelförmige Form oder Kontur auf. Sowohl der dem Arm 5 zugewandte Querschnittsabschnitt 1 des Manschettenanschlusses 300 als auch der vom Arm 5 abgewandte Querschnittsabschnitt 3 des Manschettenanschlusses 300 weisen eine in Bezug auf den Arm 5 konkave Krümmung auf. Ein Außendurchmesser des Manschettenanschlusses 300, d.h. eine Wölbung des dem Arm 5 zugewandten Querschnittsabschnitts 1, kann beispielsweise einen Radius von 0,5 bis 2 cm aufweisen.

Die erfindungsgemäße Blutdruckmanschette 200 kann hergestellt werden, indem der Manschettenanschluss 300 zwischen zwei Folien 10a und 10b eingelegt und mit diesen, z.B. mittels eines Hochfrequenz - Schweißverfahrens, verschweißt wird. Die beiden Folien 10a, 10b bilden ein Inneres 11 (in Fig. 5 nicht zu sehen), welches zum Messen des Blutdrucks mit Druck beaufschlagt werden kann.

Einer oder beide der Querschnittsabschnitt 1, 3 und/oder andere Querschnittsabschnitte des erfindungsgemäßen Schlauchs bzw. des Manschettenanschlusses 300 können alternativ auch Teil der Folie 10a und/oder 10b sein, also integraler Bestandteil hiervon.

Die Querschnittsabschnitte 1 und 3 legen ein gasdichtes Inneres 11 der Blutdruckmanschette 200 fest. Mittels des Inneren 11 wird die Arterie 7 beim Messen des Blutdrucks komprimiert. Das Innere 11 ist hierbei derart in der Blutdruckmanschette 200 vorgesehen, dass es nach Anlegen derselben an der Extremität typischerweise nicht über deren gesamten Umfang anliegt. Entsprechend nimmt das Innere 11, welches auch als eine Blase bezeichnet werden kann, bei großen Schwankungen zwischen verschiedenen Blutdruckmanschetten, etwa die Hälfte der Länge der Blutdruckmanschette 200 ein, wie dies in Fig. 5 zu erkennen ist.

Fig. 5 zeigt eine Draufsicht auf die erfindungsgemäße Blutdruckmanschette 200 in einem ersten Zustand des Nicht-Gebrauchens.

Der erste Zustand des Nicht-Gebrauchens kann z.B. ein solcher sein, in welchem die Blutdruckmanschette 200, wie in Fig. 5 gezeigt ist, flach ausgerollt vor einem Betrachter liegt.

In der in Fig. 5 gezeigten Darstellung ist der Manschettenanschluss 300, welcher sich im Inneren 11 der Blutdruckmanschette 200 befindet, nicht zu sehen. Seine Lage innerhalb des Inneren 11 ist jedoch durch die in Fig. 5 eingezeichneten Falten sowie die Strichliniendarstellung angedeutet.

An einem anderen Ende, dem - bezogen auf die Darstellung in Fig. 5 - rechten Ende, ist ein Klettverschluss 13 vorgesehen, welcher zum Verschließen der Blutdruckmanschette 200 um die Extremität dient.

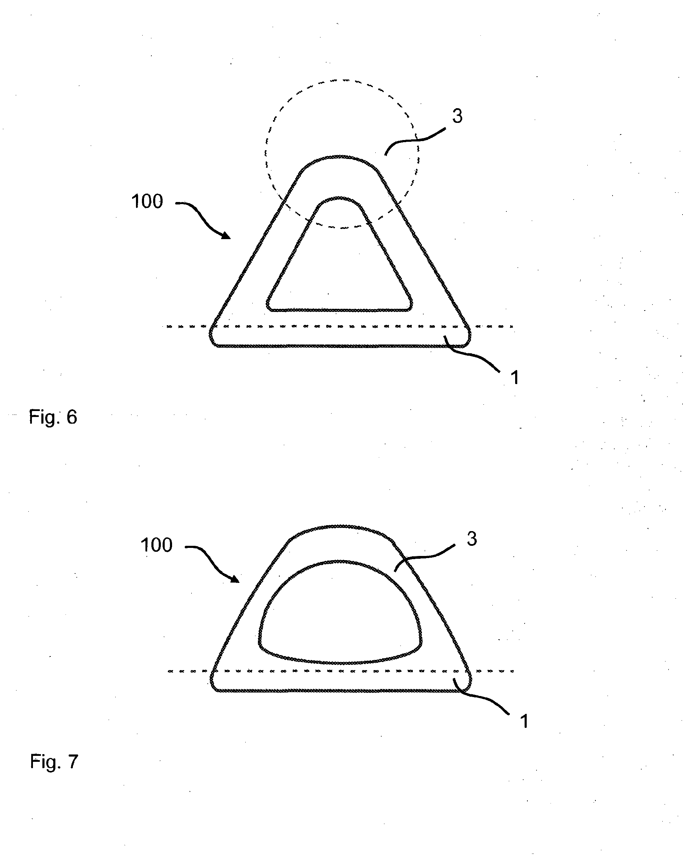

Fig. 6 zeigt einen Querschnitt eines erfindungsgemäßen Schlauchs in einem ersten Zustand des Nicht-Gebrauchens gemäß einer dritten Ausführungsform, bei welcher der Querschnitt gleichschenkelig oder im Wesentlichen gleichschenkelig ist. Eine mehr oder weniger dreieckige Form eines Querschnitts eines erfindungsgemäßen Schlauchs in einem ersten Zustand des Nicht-Gebrauchens ist in Fig. 7 für eine vierte Ausführungsform dargestellt.

### BEZUGSZEICHENLISTE

- 100: Schlauch
- 200: Blutdruckmanschette
- 300: Manschettenanschluss
- 1: Querschnittsabschnitt (der Extremität zugewandt)
- 3: Querschnittsabschnitt (der Extremität abgewandt)
- 5: Arm
- 7: Arterie
- 9: Oberarmknochen
- 10a: Folie
- 10b: Folie
- 11: Inneres der Blutdruckmanschette
- 13: Klettverschluss

## Patentansprüche

1. Kombination aus einem Schlauch (100) und einer Blutdruckmanschette (200) zum Messen eines Blutdrucks an einer Extremität eines Patienten,
wobei ein Endabschnitt des Schlauchs mit der Blutdruckmanschette verbunden ist,
wobei der Endabschnitt in wenigstens einem Querschnitt wenigstens zwei unterschiedlich gekrümmte Querschnittsabschnitte (1, 3) aufweist,
**dadurch gekennzeichnet,**
**dass** der Endabschnitt in einem zweiten Zustand des Messens, in dem er in seinem Inneren mit Druck beaufschlagt ist, einen beim Messen der Extremität zugewandten Querschnittsabschnitt mit einer Krümmung von im Wesentlichen oder gleich 0 Grad oder eine, bezogen auf die Extremität, konkave Krümmung aufweist und
**dass** sich die unterschiedlichen Krümmungen auf die Krümmungen der Außenflächen der Querschnittsabschnitte (1, 3) beziehen.

2. Kombination aus einem Schlauch (100) und einer Blutdruckmanschette (200) nach Anspruch 1, wobei sich die unterschiedlichen Krümmungen zusätzlich auf die Krümmungen der Innenflächen der Querschnittsabschnitte (1, 3) beziehen.

3. Kombination aus einem Schlauch (100) und einer Blutdruckmanschette (200) nach Anspruch 1 oder 2, wobei die Querschnittsabschnitte (1, 3) sowohl in einem ersten Zustand des Nicht-Gebrauchens als auch in dem zweiten Zustand des Messens unterschiedlich gekrümmt sind.

4. Kombination aus einem Schlauch (100) und einer Blutdruckmanschette (200) nach einem der Ansprüche 1 bis 3, wobei ein beim Messen der Extremität zugewandter Querschnittsabschnitt (1) des Schlauchs (100) im ersten Zustand eine Krümmung von im Wesentlichen oder gleich 0 Grad oder eine, bezogen auf die Extremität, konkave Krümmung aufweist.

5. Kombination aus einem Schlauch (100) und einer Blutdruckmanschette (200) nach einem der vorangegangenen Ansprüche, wobei der Schlauch (100) im ersten Zustand des Nicht-Messens einen geschlossenen, im Wesentlichen halbkreisförmigen Querschnitt aufweist.

6. Kombination aus einem Schlauch (100) und einer Blutdruckmanschette (200) nach einem der vorangegangenen Ansprüche, wobei der Querschnitt eine dreieckige oder mehreckige Form, insbesondere eine gleichschenkelige oder gleichwinklige Form, aufweist.

7. Kombination aus einem Schlauch (100) und einer Blutdruckmanschette (200) nach einem der vorangegangenen Ansprüche, wobei die konkave Krümmung im zweiten Zustand des Messens einen Radius r in einem Bereich von r zwischen einschließlich 0,5 cm bis einschließlich 2 cm mit Mittelpunkt im Bereich der Extremität aufweist.

8. Kombination aus einem Schlauch (100) und einer Blutdruckmanschette (200) nach einem der vorangegangenen Ansprüche, wobei der Schlauch (100) im zweiten Zustand des Messens einen, bezogen auf die Extremität, sichelförmigen, geschlossenen Querschnitt aufweist.

9. Kombination aus einem Schlauch (100) und einer Blutdruckmanschette (200) nach einem der vorangegangenen Ansprüche, wobei ein einem äußeren Umfang der Extremität des Patienten im zweiten Zustand des Messens zugewandter Querschnittsabschnitt (1) eine der Extremität zugewandte konkave Krümmung in einem Bereich von 0 bis 1/r aufweist, wobei r jeweils der Radius eines im Bereich eines Abschnitts des äußeren Umfangs der Extremität angelegten Schmiegekreises ist.

10. Kombination aus einem Schlauch (100) und einer Blutdruckmanschette (200) nach einem der vorangegangenen Ansprüche, wobei der Schlauch (100) aus TPR 60 ShA ausgestaltet ist oder wenigstens abschnittsweise TPR 60 ShA aufweist.

11. Kombination aus einem Schlauch (100) und einer Blutdruckmanschette (200) nach einem der vorangegangenen Ansprüche, wobei der Endabschnitt als Manschettenanschluss (300) ausgestaltet ist, welcher vorgesehen ist, zum Messen des Blutdrucks wenigstens abschnittsweise oder teilweise oder vollständig in einem Inneren der Blutdruckmanschette (200) angeordnet zu sein.

12. Kombination aus einem Schlauch (100) und einer Blutdruckmanschette (200) nach einem der vorangegangenen Ansprüche, wobei die Blutdruckmanschette (200) wenigstens eine erste Folie (10a) und eine zweite Folie (10b) aufweist, wobei der Schlauch (100) im Bereich des Manschettenanschlusses (300) wenigstens abschnittsweise zwischen die erste Folie (10a) und die zweite Folie (10b) der Blutdruckmanschette (200) eingeschweißt ist.

## Claims

1. A combination of a tube (100) and a sphygmomanometer cuff (200) for measuring blood pressure at an extremity of a patient,
wherein an end segment of the tube is connected to the sphygmomanometer cuff,
wherein the end segment has, in at least one cross-section, at least two differently curved cross-sectional segments (1, 3),
**characterised in that**
the end segment, in a second state of measurement in which pressure is applied to it in its interior, has a cross-sectional segment, facing the extremity during the measurement, with a curvature of substantially 0 degrees or equal to 0 degrees or a concave curvature relative to the extremity, and
the different curvatures relate to the curvatures of the outer faces of the cross-sectional segments (1, 3).

2. A combination of a tube (100) and a sphygmomanometer cuff (200) according to claim 1, wherein the different curvatures additionally relate to the curvatures of the inner faces of the cross-sectional segments (1, 3).

3. A combination of a tube (100) and a sphygmomanometer cuff (200) according to claim 1 or 2, wherein the cross-sectional segments (1, 3) are curved differently both in a first state of non-use and in the second state of measurement.

4. A combination of a tube (100) and a sphygmomanometer cuff (200) according to one of claims 1 to 3, wherein a cross-sectional segment (1) of the tube (100) facing the extremity during the measurement has, in the first state, a curvature of substantially 0 degrees or equal to 0 degrees or a concave curvature relative to the extremity.

5. A combination of a tube (100) and a sphygmomanometer cuff (200) according to one of the preceding claims, wherein the tube (100) has, in the first state of non-measurement, a closed, substantially semicircular cross-section.

6. A combination of a tube (100) and a sphygmomanometer cuff (200) according to one of the preceding claims, wherein the cross-section has a triangular or polygonal form, in particular an equilateral or equiangular form.

7. A combination of a tube (100) and a sphygmomanometer cuff (200) according to one of the preceding claims, wherein the concave curvature has, in the second state of measurement, a radius r in a range of r between inclusively 0.5 cm to inclusively 2 cm with a central point in the region of the extremity.

8. A combination of a tube (100) and a sphygmomanometer cuff (200) according to one of the preceding claims, wherein the tube (100) has, in the second state of measurement, a sickle-shaped closed cross-section relative to the extremity.

9. A combination of a tube (100) and a sphygmomanometer cuff (200) according to one of the preceding claims, wherein a cross-sectional segment (1) facing an outer periphery of the extremity of the patient in the second state of measurement has a concave curvature facing the extremity in a range of 0 to 1/r, wherein r in each case is the radius of an osculating circle applied in the region of a segment of the outer periphery of the extremity.

10. A combination of a tube (100) and a sphygmomanometer cuff (200) according to one of the preceding claims, wherein the tube (100) is configured from TPR 60 ShA or at least in segments has TPR 60 ShA.

11. A combination of a tube (100) and a sphygmomanometer cuff (200) according to one of the preceding claims, wherein the end segment is configured as a cuff connection (300) which, for measurement of the blood pressure, is provided so as to be arranged at least in segments or in part or completely in an interior of the sphygmomanometer cuff (200).

12. A combination of a tube (100) and a sphygmomanometer cuff (200) according to one of the preceding claims, wherein the sphygmomanometer cuff (200) has at least a first film (10a) and a second film (10b), wherein the tube (100) in the region of the cuff connection (300) is welded at least in segments between the first film (10a) and the second film (10b) of the sphygmomanometer cuff (200).

## Revendications

1. Combinaison d'un tuyau (100) et d'un brassard pneumatique (200) pour la mesure d'une pression artérielle à une extrémité d'un patient,
dans laquelle une section d'extrémité du tuyau est reliée au brassard pneumatique,
dans laquelle la section d'extrémité présente dans au moins une section transversale au moins deux sections de section transversale de courbure différente (1, 3),
**caractérisée en ce**
**que** la section d'extrémité présente dans un second état de la mesure, dans lequel elle est sollicitée en pression dans sa partie intérieure, une section de section transversale tournée vers l'extrémité lors de la mesure avec une courbure d'essentiellement ou égale à 0 degré ou une courbure concave par rapport à l'extrémité et
**que** les différentes courbures se rapportent aux courbures des surfaces externes des sections de section transversale (1, 3).

2. Combinaison d'un tuyau (100) et d'un brassard pneumatique (200) selon la revendication 1, dans laquelle les différentes courbures se rapportent en outre aux courbures des surfaces internes des sections de section transversale (1, 3).

3. Combinaison d'un tuyau (100) et d'un brassard pneumatique (200) selon la revendication 1 ou 2, dans laquelle les sections de section transversale (1, 3) sont courbées différemment aussi bien dans un premier état de non-utilisation que dans le second état de la mesure.

4. Combinaison d'un tuyau (100) et d'un brassard pneumatique (200) selon une des revendications 1 à 3, dans laquelle une section de section transversale (1) du tuyau (100) tournée vers l'extrémité lors de la mesure présente dans le premier état une courbure d'essentiellement ou égale à 0 degré ou une courbure concave par rapport à l'extrémité.

5. Combinaison d'un tuyau (100) et d'un brassard pneumatique (200) selon une des revendications précédentes, dans laquelle le tuyau (100) présente dans le premier état de non-mesure une section transversale fermée, essentiellement semi-circulaire.

6. Combinaison d'un tuyau (100) et d'un brassard pneumatique (200) selon une des revendications précédentes, dans laquelle la section transversale présente une forme triangulaire ou polygonale, notamment une forme isocèle ou équilatérale.

7. Combinaison d'un tuyau (100) et d'un brassard pneumatique (200) selon une des revendications précédentes, dans laquelle la courbure concave présente dans le second état de la mesure un rayon r dans une plage de r comprise entre 0,5 cm inclus jusqu'à 2 cm inclus avec un centre dans la région de l'extrémité.

8. Combinaison d'un tuyau (100) et d'un brassard pneumatique (200) selon une des revendications précédentes, dans laquelle le tuyau (100) présente dans le second état de la mesure une section transversale fermée, en forme de croissant, par rapport à l'extrémité.

9. Combinaison d'un tuyau (100) et d'un brassard pneumatique (200) selon une des revendications précédentes, dans laquelle une section de section transversale (1) tournée vers une périphérie extérieure de l'extrémité du patient dans le second état de la mesure présente une courbure concave tournée vers l'extrémité dans une plage de 0 à 1/r, dans laquelle r est à chaque fois le rayon d'un cercle osculateur appliqué dans la région d'une section de la périphérie extérieure de l'extrémité.

10. Combinaison d'un tuyau (100) et d'un brassard pneumatique (200) selon une des revendications précédentes, dans laquelle le tuyau (100) est configuré en TPR 60 ShA ou présente au moins par section du TPR 60 ShA.

11. Combinaison d'un tuyau (100) et d'un brassard pneumatique (200) selon une des revendications précédentes, dans laquelle la section d'extrémité est configurée comme raccord de brassard (300) qui est prévu d'être disposé au moins par section ou partiellement ou entièrement dans une partie intérieure du brassard pneumatique (200) pour la mesure de la pression artérielle.

12. Combinaison d'un tuyau (100) et d'un brassard pneumatique (200) selon une des revendications précédentes, dans laquelle le brassard pneumatique (200) présente au moins un premier film (10a) et un second film (10b) dans laquelle le tuyau (100) est soudé dans la région du raccord de brassard (300) au moins par section entre le premier film (10a) et le second film (10b) du brassard pneumatique (200).
